(19) Europäisches Patentamt
European Patent Office
Office européen des brevets

(11) Publication number: **0 492 823 A2**

# (12) EUROPEAN PATENT APPLICATION

(21) Application number: **91311190.2**

(22) Date of filing: **02.12.91**

(51) Int. Cl.[5]: **C07D 265/06**, C07D 263/04, C07D 307/22, C07D 309/14

(30) Priority: **26.12.90 US 634006**

(43) Date of publication of application: **01.07.92 Bulletin 92/27**

(84) Designated Contracting States: **DE FR GB IT**

(71) Applicant: **MINNESOTA MINING AND MANUFACTURING COMPANY**
**3M Center, P.O. Box 33427**
**St. Paul, Minnesota 55133-3427(US)**

(72) Inventor: **Moore, George G. I., c/o Minnesota Mining and**
**Manufact. Co., 2501 Hudson Road, P.O. Box 33427**
**St. Paul, Minnesota 55133-3427(US)**
Inventor: **Hansen, John C., c/o Minnesota Mining and**
**Manufact. Co., 2501 Hudson Road, P.O. Box 33427**
**St. Paul, Minnesota 55133-3427(US)**

(74) Representative: **Baillie, Iain Cameron et al**
**Ladas & Parry Altheimer Eck 2**
**W-8000 München 2(DE)**

## (54) Perfluorinated t-amino cyclic ethers.

(57) Perfluorinated, t-amino cyclic ethers are provided. Electrochemical fluorination of N,N-dialkyl carboxamides provides Class 1 compounds of one or a mixture of perfluorinated, N-alkyl, aliphatic-substituted, saturated 1,3-oxazolidine and perfluorinated, N-alkyl, aliphatic-substituted, saturated 1,3-oxazine. Class 2 compounds comprise one or a mixture of Class 1 compounds, as well as perfluorinated, aliphatic-substituted 2-dialkylaminotetrahydrofuran and perfluorinated, aliphatic-substituted 2-dialkylamino dihydropyran.

EP 0 492 823 A2

This invention relates to liquid, perfluorinated, amino, cyclic ethers; their acids, amide, ester and dihydroalcohol derivatives, and to their preparation, and to several uses of the liquid, perfluorinated, amino, cyclic ethers, including, as heat transfer fluid in vapor phase (or condensation) heating processes, or heat removal.

The development of mass soldering techniques has played an important role in the phenomenal growth of the electronics industry. A significant milestone in the advancement of this technology was the introduction in 1975 of vapor phase (or condensation) heating in soldering operations by Western Electric Company, Inc., see, for example, U.S. Patents Re 30,399 (Ammann et al.) and 4,238,186 (Pfahl); Chu et al., Condensation Soldering: A New Mass Soldering Process, Welding Journal, April 1975; and Karpel, Mass-Soldering Equipment For The Electronic Industry, Quarterly Journal of the International Tin Research Industry Institute, 1, 130, 1981.

Briefly, the vapor phase soldering process comprises, first, heating a pool of an inert perfluorinated liquid to boiling in a tank. A fluorocarbon vapor layer forms above the liquid, and the temperature of the dense vapor is the same as the boiling point of the liquid itself. With solder already in place, the workpiece to be soldered is lowered into the vapor zone, where the vapors condense on the relatively cool workpiece. For example, the workpiece may he a printed circuit board with pre-applied solder or soldered preforms. Immersing the relatively cool workpiece enables rapid transference of the vapor's latent heat of vaporization (or condensation) to the workpiece, causing the solder to reflow. Once the solder has reflowed, the soldered workpiece is removed from the vapor.

The vapor phase soldering process was first commercialized when Minnesota Mining and Manufacturing Company ("3M") provided a product called Fluorinert™ Electronic Liquid FC-70, a perfluoroalkyl tertiary monoamine liquid boiling at about 215°C. FC-70 is a heat transfer fluid used for vapor phase soldering.

Inert perfluorinated liquids have also proved successful as vapor phase condensation heating fluids for curing a solder mask. The curing process as described in 3M's Technical Bulletin, "Fluorinert™ Electronic Liquids Vapor Phase Condensation Heating for Curing of Solder Mask," No. 98-0211-2327-2(46.3)R1, uses Fluorinert™ Electronic Liquid FC-43, a perfluorinated liquid boiling at about 174°C.

The 3M product, Fluorinert™, is made by an electrochemical fluorination (ECF) process. The hydrocarbon precursor is electrolyzed with anhydrous hydrogen fluoride. This is an electrochemical reaction in which carbon-bound hydrogen atoms are replaced with fluorine atoms. A more thermally stable product of this type is sold by 3M as Fluorinert™ Electronic Liquid FC-5312. These products and the vapor phase condensation heating technology are described by R.D. Danielson in "Vapor-Phase Soldering with Perfluorinated Inert Fluids," Proceedings of Technical Program NEPCON, 1979, Anaheim, California and in 3M's Technical Bulletin, "Fluorinert™ Liquids Vapor Phase Heating References," No. 98-0211-43775(58.5) BG, issued May, 1988. ("Fluorinert" and "FC" are trademarks of 3M).

The use of various condensation heating fluids are described in the above-cited references. However, those fluids do not meet all the criteria indicative of a thermally stable inert heat transfer fluid. As the area of vapor phase soldering has developed, need has arisen for a wide range of fluids for production of a wider range of temperatures. Additionally, when used under some conditions, perfluoroisobutylene ("pfib") and acidic products, for example, hydrogen fluoride (HF), a particularly corrosive acid to glass or any silicon containing materials, are formed. Additionally, electrochemical fluorination ("ECF") can result in rearrangement and fragmentation products. Certain minor by-products of ECF can be decomposed under use conditions, creating conditions potentially toxic and hazardous to the user.

The pfib and HF decomposition products are formed in trace amounts during the vaporization (condensation) cycle of vapor phase soldering techniques. Concerns about these toxic compounds are described in Electronic Production, July, 1985, pp. 47, 49, and in 3M Technical Bulletin No. 98-0211-4411-2(78.2) R1 XY, issued June, 1988.

Safety-related and corrosion-related issues have led to the need for heat transfer fluids that produce lower amounts of pfib and HF during vapor phase soldering. The use of perfluorotetradecahydrophenanthrene, as a heat transfer fluid is disclosed in U.S. Patent No. 4,549,686, Sargent et al. and has been represented as not producing detectable amounts of pfib and HF.

Briefly, in one aspect of this invention, a first class of novel, perfluorinated, tertiary amino, cyclic ethers, which are normally liquid, is provided, each such liquid (referred to herein as Class 1 liquids) comprising one or a mixture of (a) perfluorinated, N-alkyl, aliphatic-substituted, saturated 1,3-oxazolidine and (b) perfluorinated, N-alkyl, aliphatic-substituted, saturated 1,3-oxazine.

An example of a liquid of said Class 1 is that comprising one or a mixture of the following:

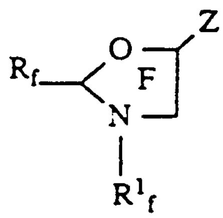

(I)

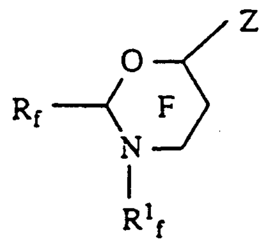

(II)

The fluorinated aliphatic portion of the liquids, depicted as $R_f$ in Formulas I and II, is generally a saturated fluoroaliphatic group having a chain of one or more fully-fluorinated, saturated-aliphatic carbon atoms. The skeletal chain of $R_f$ can be straight, branched, or, if sufficiently large, cyclic or contain a cyclic structure. It can be composed of only carbon and fluorine, or it can include carbon, fluorine and a catenary divalent oxygen, or a caternary trivalent nitrogen, such hetero atom being a chemically stable linkage between the fluorocarbon portions of the fluoroaliphatic group. Generally, $R_f$ will have 1 to 20 carbon atoms. A preferred embodiment of $R_f$ is a perfluorinated, saturated, cycloaliphatic such as one having 4, 5, or 6 ring carbon atoms, e.g., a perfluorocyclohexyl, $C_6F_{11}$- or $CF_3C_6F_{10}$-, a ring carbon atom of which is covalently bonded to the ring carbon atom positioned between the oxygen and nitrogen of the ring depicted in Formula I or II.

Z is a lower fluoroalkyl having 1 to 8 carbon atoms or a fluorine atom. The $R^1_f$ attached to the depicted tertiary amino ring atom comprises a fully-fluorinated, saturated-aliphatic carbon atom, or a cyclic or acyclic group comprising a plurality of such carbon atoms, preferably in a chain that can be a straight (normal) chain or a branched chain, e.g., $-(CF_2)_3$ or $-CF(CF_3)CF_3$. $R^1_f$ and Z taken together can form a 5-, 6-, or 7-membered ring composed of carbon atoms only, or composed of carbon atoms and N or O hetero atoms. Further, $R^1_f$ and $R_f$ taken together can form a 5-, 6-, or 7-membered ring composed of carbon atoms only, or composed of carbon atoms and N or O hetero atoms.

Unless otherwise stated or apparent, the term "perfluorinated" means that all or essentially all of the hydrogen atoms in an organic compound are replaced by fluorine atoms. The symbol "F" in a ring denotes the ring is a saturated perfluorinated ring.

In another aspect of the present invention, a second class of novel, perfluorinated, tertiary amino, cyclic ether, which is normally liquid is provided, each such liquid (referred to herein as Class 2 liquids) comprising one or a mixture of (a) perfluorinated N-alkyl, aliphatic-substituted, saturated 1,3-oxazolidine, (b) perfluorinated, N-alkyl, aliphatic-substituted, saturated 1,3-oxazine, (c) perfluorinated, aliphatic-substituted 2-dialkylaminotetrahydrofuran and (d) perfluorinated, aliphatic-substituted 2-dialkylaminodihydropyran.

An example of a liquid of said Class 2 is that comprising a mixture of the following:

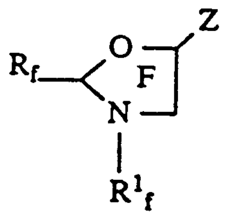

(I)

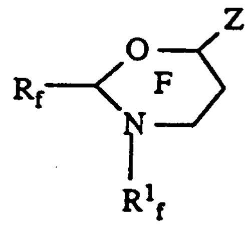

(II)

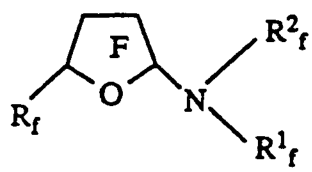

(III)

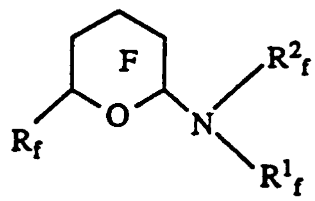

(IV)

$R_f$, $R^1_f$, and Z are as defined in Formulas I and II, hereinabove. $R^2_f$ is defined as $R^1_f$ and may be the same or different, with the proviso that if either $R^1_f$ or $R^2_f$ is a trifluoromethyl group, the sum of carbon atoms of $R^1_f$ and $R^2_f$ comprise at least 3 carbon atoms. Further, $R^1_f$ and $R^2_f$ taken together can form a 5-, 6-, or 7-membered ring composed of carbon atoms only, or composed of carbon atoms and N or O hetero atoms.

In still another aspect of the invention, a method of making said perfluorinated, amino, cyclic ethers (for brevity sometimes hereinafter referred to as perfluorinated liquids) is provided. The perfluorinated liquids of the present invention may be prepared by electrochemical fluorination (ECF) of hydrocarbon or partially fluorinated precursors. The precursors used in the electrochemical fluorination process as a cell feed are perfluorinatable N,N-dialkyl carboxamides that are described in the literature or commercially available, unless otherwise stated or apparent.

An example of a perfluorinatable precursor used as the cell feed in the present invention is as follows:

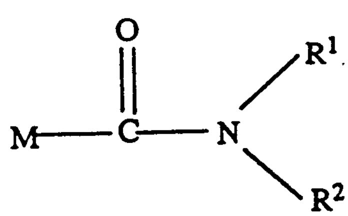

(V)

M is either R or

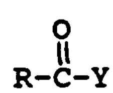

wherein R is an acyclic, cyclic, aromatic, fluoroaliphatic, fluorocycloaliphatic group having one or more fluorinatable carbon atoms and is composed of only carbon, hydrogen, and fluorine, or carbon and hydrogen and can include caternary divalent oxygen or caternary trivalent nitrogen, such hetero atoms being a chemically stable linkage between the carbon portion of the R group; and

Y is selected from

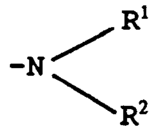 ,

-X, -OH and $-OR^3$

wherein X is a halide, preferably fluorine, and

$R^3$ is a lower alkyl having 1 to 8 carbon atoms; and

$R^1$ or $R^2$ is an alkyl group with at least 2 carbon atoms and generally not more than 8 carbons.

$R^1$ and $R^2$ may be the same or different, with a first proviso that if either $R^1$ or $R^2$ is a trifluoromethyl group, the sum of the carbon atoms of $R^1$ and $R^2$ comprise at least 3 carbon atoms and a second proviso that if either $R^1$ or $R^2$ is an aromatic group, the other is an alkyl group having 2 or more carbon atoms. Further, $R^1$ and $R^2$ taken together can form a 5-, 6-, or 7-membered ring composed of carbon atoms only, or composed of carbon atoms and N or O hetero atoms. The preferred M is a perfluorinated cyclohexyl or a substituted cyclohexyl group.

Generally, the precursors can be simply and inexpensively synthesized from secondary amines and carboxylic acids, esters or acyl chlorides, as discussed below.

The resulting perfluorinated liquids are essentially free of hydrogen, chlorine, and bromine atoms. They are advantageously chemically stable, thermally stable, non-oxidizing, electrically non-conducting, non-flammable and boil to produce vapors with the same said properties as the liquid. Those properties, together with the liquid's relatively high vapor densities and high latent heat of vaporization, make the liquids particularly useful as a heat transfer (or working) fluids for vapor phase (or condensation) heating operations, such as soldering and solder mask curing. Furthermore, the conductivity of the precursor amides is sufficiently high, that excellent cell operation is obtained without the presence of any conductivity additives, yielding novel perfluorinated liquids with less unstable fragments than the presently known perfluorinated fluids. Thus, when used as a heat transfer fluid, the liquids produce significantly lower amounts of pfib and HF byproducts. As a result, higher yields, lower processing costs, and safer products are obtained.

The perfluorinated liquids also have relatively low pour points, wide liquid ranges, and relatively low viscosities, all of which facilitate their handling and use over wide temperature ranges.

The perfluorinated liquids of this invention can be prepared by electrochemical fluorination. The "Simons process" or the "Simons electrochemical fluorination process" is a known, commercially practiced fluorination process carried out in an electrolytic cell (commonly referred to as a "Simons cell") to perfluorinate certain classes of organic compounds. The Simons process is carried out in an electrolytic solution of the organic feed in essentially anhydrous liquid hydrogen fluoride. An early patent describing such technology is U.S. Patent No. 2,519,983 (Simons), which contains a drawing of a Simons cell and its appurtenances A description and photographs of laboratory and pilot plant cells appear at pages 416-418 of Vol. 1 of "Fluorine Chemistry," edited by J. H. Simons, published in 1950 by Academic Press, Inc., New York. Further details on the Simons process will be omitted in the interest of brevity and the above-referenced patent in incorporated herein by reference for such details.

The "Simons process" is a known type of highly energetic reaction that is often accompanied by carbon-carbon bond cleavage. This carbon-carbon bond cleavage leads to fragmentation and recombination phenomena, often resulting in the formation of low and high molecular weight by-products. Those by-products include tars that often deposit on the electrodes of the electrochemical cell and impede current flow, with corresponding increase in the voltage necessary to continue the electrochemical reaction. The increase in voltage results in increased fragmentation and eventual current stoppage and termination of the electrical fluorination reaction. These problems become aggravated when the number of carbon atoms in the compound used as the cell feed, that is, the organic substance being fluorinated, is relatively high, for example, more than 12 carbon atoms.

Surprisingly and notwithstanding the relatively high number of carbon atoms, namely 12 to 20 carbon atoms and more, in the precursors used in the Simons process for the perfluorinated liquids of this invention, the perfluorinated liquids can be produced by ECF at relatively low voltage and with relatively low amounts of by-products. Use as a fluid for vapor-phase heating produces relatively low amounts of pfib and HF, especially in the desired boiling range. The low amount of by-products is further enhanced by the sufficient conductivity of the amides used as precursor cell feeds.

Reviews and literature cited therein, such as that by I.N. Rozkov, Org. Electrochem., 2nd ed, 1983, p805ff disclose that precursor amides undergo cleavage during ECF to form acid fluorides. Such amide

cleavage limits the applicability of ECF as a fluorination technique. Therefore, the cyclization that occurs in the ECF process as used in the present invention is unexpected in light of the literature examples of cleavage.

Advantageously, if Rozkov-type cleavage is observed, relatively well-defined by-products are produced. Referring to Formula V, cleavage will generally occur at the C-N bond, essentially splitting the precursor molecule in half. By-products with molecular weights of approximately half that of the precursor are easily removed. This is of interest, since the boiling point for the novel perfluorinated liquid depends, in part, on the amount of by-products still present, in addition to the carbon content of the product.

The preferred ECF method of the invention is to electrolyze the precursor feed without the aid of a conductivity additive, since the conductivity of the precursor organic feed used in the present invention is typically adequate. However, if desired or to increase conductivity, a conductivity additive may be used in an amount of 1 to 20 percent of the weight of the precursor feed. For example, additives such as sodium fluoride, acetic anhydride, an organic sulfur containing compound as described in U.S. Patent Nos. 3,028,321 (Danielson), 3,692,643 (Holland) and 4,739,103 (Hansen), Japanese Patent No. 88030399 (Daikin Kogyo KK.) or any other additive known to those skilled in the art may be used and still remain within the scope and principles of the present invention.

The major products of interest can be separated from the crude cell product by simple distillation. In general, the distillate will contain a mixture of major products. The number of major products present is dependent on the starting precursor skeletal structure. The balance of materials in the distillate will be partially fluorinated compounds, and other perfluorinated compounds including decomposition by-products, structural isomers, and higher or lower molecular weight fragments.

After the crude perfluorinated liquid is produced, it is removed from the cell. It can then be treated to remove any residual HF by-product, for example by treating the crude cell product with NaF. The crude product is then fractionally distilled in one or more steps and treated with caustic, for example, as described in U.S. Patent No. 4,788,339 (Moore et al.) and incorporated herein by reference for such procedure. A working example is hereinafter set forth of the preparation of the perfluorinated liquids of this invention.

The perfluorinated liquids of this invention can be used in the vapor phase heating of an article. In such a process, a fluorinated liquid is boiled to produce a saturated vapor which contacts the article to be heated, the vapor condensing on the relatively cool surface of the article and raising its temperature to achieve a desired purpose, for example, to cure a polymeric or resinous protective coating on an article, for example an optical lens.

In using the perfluorinated liquids as a heat transfer fluid in vapor phase (or condensation) soldering, the manipulative steps and equipment therefor can be the same as that used with a fluorinated liquid such as Fluorinert™ Liquids FC-70, described in 3M Technical Bulletin No. 98-0211-4377-5(58.5)BE. The described process comprised heating in a tank of a soldering system a pool or body, one of the perfluorinated liquids of this invention to its boiling point to form and maintain a body or zone of saturated vapor thereof at the same temperature in the space between the boiling liquid and condensing means such as cooling coils, immersing in said body of vapor, a relatively cool workpiece assembly to be soldered, allowing the vapor to condense on the workpiece assembly and give up its latent heat of vaporization so as to raise the solder to the temperature of the perfluorinated liquid and cause melting and reflow of the solder, and then removing from the soldering system the workpiece with its resulting solder joints. The reflow of the solder takes place in the vapor zone of the soldering system since the temperature of the vapor is higher than the melting point of the solder but not so high that the workpiece will be damaged.

The solder can be pre-applied to the workpiece in the common ways now known and used in mass reflow soldering operations. Since the vapor phase soldering environment is essentially oxygen-free, a less active flux can be used or, in some cases, not at all.

The soldering system used with the perfluorinated liquids can be either a batch system or a continuous-in-line system. For batch soldering, workpiece assemblies are lowered vertically into the saturated vapor zone. A secondary vapor blanket may be used to help contain the primary vapor in the unit. In-line vapor phase soldering uses a continuous conveyer to transport assemblies horizontally through the saturated vapor zone. U.S. Patent Re. 30,399 illustrates equipment that can be used in batch and continuous systems. There are a number of liquids commercially available that can be used to provide secondary vapors, such as 3M SF-2 Secondary Fluid described in 3M's bulletin 98-0211-4542-2, issued March, 1989.

In batch soldering processes, the reflow time is dependent on the surface area and mass of an assembly and should be determined for each application. In typical situations, reflow time will range from approximately 15 to 30 seconds. For a massive workpiece, 60 to 90 seconds are not unusual. For in-line systems, reflow time is dependent on the mass of the workpiece. Typical reflow times for small pieces range from 10 to 20 seconds.

There is a host of electronic assemblies that can be soldered by vapor phase heating and such assemblies can likewise be soldered in the practice of this invention. Some of the assemblies include wire-wrap pin backplane assemblies, printed circuit boards to which Sn/Pb or Sn electroplate is to be fused, terminal configurations that incorporate Sn plating on small components, assemblies involving odd geometries or dissimilar materials such as gyro stator housing, and various devices, board types, and configurations including lead lists and leaded packages, plastic and ceramic devices, ceramic or organic boards, and single or double-sided assemblies that have planar mounting surfaces.

Vapor phase heating equipment that can be used with the perfluorinated liquids is available from several manufacturers. All the equipment share the commonality of a heat source, such as an immersion heater or thermal mass heater, to generate the vapor zone. A representative example of a production system for vapor phase soldering is the "Phase-Four" production system supplied by Hybrid Technology Corporation ("HTC") and described in technical bulletin (undated).

Though the foregoing description focuses on the use of the perfluorinated liquids of this invention in vapor phase soldering operations, such liquids can be used as heat transfer medium in other vapor phase heating
applications where a rapid heat transfer is advantageous, such as in the processing of various polymers, epoxies, metals and coatings, for example, in the curing of thermosetting resin coatings. Liquids of this invention can be used for rapid heat removal as well.

Fluorochemicals that have a functional moiety can exhibit surface-active properties that make them useful as surface-active agents (or surfactants). Surfactants are useful in improving or imparting properties to aqueous and non-aqueous (organic) liquid systems such as wetting, penetration, spreading, leveling, foaming, foam stabilization, flow properties, emulsification, dispersability and oil, water, and soil repellency. Furthermore, such fluorochemicals are useful as surface treating agents (e.g., in the form of coatings) in modifying surface properties of substrates, such as fibrous textiles, leather, and paper, for example in modifying their water and oil repellancy.

The perfluorinated liquids of this invention can be made by electrochemical fluorination of precursors, which are preferably carboxamides. The precursors can be synthesized from methods well-known in the art, for example see Advanced Organic Chemistry, March, 3rd ed. (Wiley & Sons) 1985, such as the acylation of amines, using secondary amines and carboxylic acids, esters or acyl halides.

Furthermore, any methods or processes for synthesizing amides known to those skilled in the art can be used and still be within the spirit and principles of the present invention.

Useful precursor compounds for conversion to the perfluorinated liquids of this invention by electrochemical fluorination include, but are not limited to the following compounds:

Alkyl Amides

N,N-dipropylbutyramide
N,N-dipropyloctanamide
N,N,N,N-tetrapropyl-1,6-hexanedicarboxamide
N,N-dipentyl-1-decanecarboxamide
N,N-dibutyl-1-decanecarboxamide
N-methyl-N-propyl decanecarboxamide

Cyclic Amides

N-benzoylpiperidine
N-(perfluorobutyryl)piperidine
N-propylcaprolactam
N-octanoylmorpholine

Partially-fluorinated Amides

N,N-dipropyl perfluorocyclohexanecarboxamide
N,N-dibutyl perfluorocyclohexanecarboxamide
N,N-dipropyl perfluoro(methylcyclohexane)carboxamide
N,N-dipentyl perfluoromethylcyclohexane-carboxamide
N,N,N,N-tetrapropyl perfluoro 1,4-cyclohexane-dicarboxamide
N,N-dipentyl perfluorocyclohexanecarboxamide

Aromatic Amides

N,N,N,N-tetrapropyl terephthalamide
N,N-dipropyl p-toluamide
N,N-dibutyl benzamide

Carbonyl Amides

3-methoxycarbonyl-N,N-dipropylpropionamide

This invention is further illustrated by the following examples. However, these examples are merely for illustration purposes and in no way are to be construed to limit or otherwise restrict the scope of the present invention. All the examples used to illustrate the present invention and obtained by the electrochemical process were carried out in a Simons cell. A 50 ampere cell as described in U.S. Patent No. 2,519,983 was charged with 1500 g of liquid anhydrous hydrogen fluoride. The propyl, butyl and pentyl groups are n-propyl, n-butyl and n-pentyl groups unless otherwise indicated.

The perfluorinated liquids produced by ECF are generally complex mixtures of Class 1 compounds or Class 2 compounds depending on the precursor selected. Additionally, partially fluorinated products, and perfluorinated products such as structural isomers, and higher or lower molecular weight fragments are present. The proposed structures are based on (a) products have the same number of carbon, nitrogen and oxygen atoms as did the precursor, confirmed by boiling point and gas chromatography-mass spectrometry (GC/MS); (b) the number of fluorine atoms in the main product is identical to the hydrogen atoms in a saturated precursor, (plus fluorine atoms due to saturation of unsaturated precursors); (c) no carbonyl groups are detectable by infrared spectroscopy (IR); and (d) $^{19}F$ NMR shows no olefinic unsaturation, confirming the presence of a cyclic structure. Although features can be identified, the complexity of these spectra makes them difficult to interpret conclusively in support of 5- or 6-membered rings. For amides of linear alkanoic acids, alternative cyclization via the alkanoyl group is indicated by GC/MS fragmentation patterns.

Example 1

The electrochemical fluorination (ECF) of N,N,N,N-tetrapropyl terephthalamide was carried out in a Simons cell of the type described in U.S. Patent No. 2,713,593. The precursor was suspended in HF and fed as a slurry into the cell. The Simons cell was operated at ambient temperature and pressure, and at 6.5 volts and 20 amps/ft$^2$ average current density.

The crude perfluorinated liquid was separated from the hydrogen fluoride layer. Some of the crude perfluorinated liquid was treated with a small amount of NaF to remove residual hydrogen fluoride. The product was then fractionated. The fractionation produced a complex mixture with 43 wt.% being in the boiling point (bp) range of 220-260 °C. Redistillation produced a mixture in the range of 200-270 °C.

Analysis by $^{19}F$ NMR of the heart cut, having a bp in the range of 147-167 °C/12 Torr and having a pour point of -20 °C, indicated that the mixture contained Class 2 compounds, including the following structure, supported by GC/MS analysis (m/e 1022).

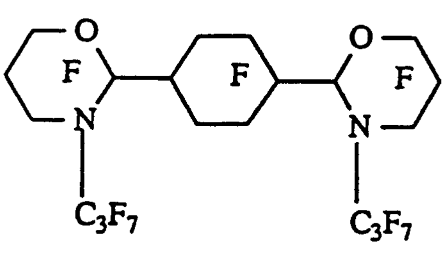

Example 2

A mixture of 450 mL (3.25 mol) of dipropylamine, 455 mL of triethylamine, and 1 L of methylene chloride was treated dropwise with 500 grams (3.23 mol) of p-toluoyl chloride. The resulting slurry was washed with water, dried and stripped. This preparation and a second preparation of the same scale were

distilled to 1286 grams (91%), with a bp of 120 °C/0.3 Torr.

The ECF of N,N-dipropyl p-toluamide was carried out in a 1500cc Simons cell, at 25 to 31 amps, 20 to 30 psig, and from 50 °C to 60 °C. The majority of product was taken off from the lower phase of the HF electrolyte. The average voltage, as measured from anode to cathode was about 7.1 volts. The crude production rate was about 63 wt.% of theoretical. The significant majority of product was in the boiling range of $C_{13}$ to $C_{15}$ as determined by gas chromatography (GC) analysis.

Distillation of the crude ECF product on a 3-plate Snyder column produced a fraction with a bp in the range of 180-200 °C and a pour point of -52 °C. The fraction contained Class 2 compounds, including the following proposed structure. GC-MS of this fraction showed a component at m/e 711, corresponding to $C_{14}F_{27}NO$.

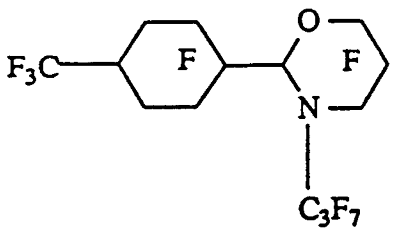

Example 3

The ECF of N,N-dibutyl benzamide was carried out in a Simons cell of 1500cc capacity using 5% dimethyl disulfide as a conductivity additive. The cell was run at an average of 30 amps and at 6.2 volts anode to cathode voltage. Cell temperature was about 60 °C and 25 to 35 psig. A total of 808 grams of organic were charged to the cell over the 180 hour run, during which time a total of 1660 grams of crude perfluorinated liquid was collected. The collected liquid contained Class 2 compounds, including the following proposed structure. By GC identification, 62% to 75% of the collected liquid was in the $C_{14}$ to $C_{16}$ boiling range.

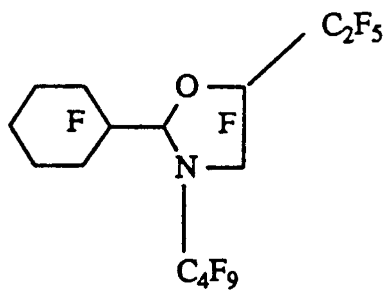

Example 4

The ECF of N,N-dipropylbutyramide was carried out in a Simons cell of 1500 cc at 35 °C and 10 psig. In the 171.4 hour run, 1114 grams of material were electrochemically fluorinated at 20 to 33 amps current.

Fractionation of the crude ECF liquid yielded a main component having a bp in the range of 137-140 °C. The component was treated with aqueous KOH and redistilled to a bp in the range of 137-144 °C and having a pour point of -90 °. The crude ECF liquid contained Class 2 compounds including the following proposed structure. GC/MS supported the major peak to be a cyclized product having the formula $C_{10}F_{21}NO$.

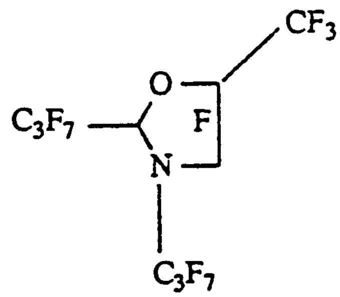

Example 5

A mixture of 275 mL (2.0 mol) of dipropylamine and 500 mL CFC-113 was treated slowly with 330 grams of the ECF product of benzoyl chloride, a mixture of at least 70% perfluorocyclohexanecarbonyl fluoride and up to 30% perfluoromethylcyclopentanecarbonyl fluorides. For brevity, the derivatives of the acyl fluoride mixture will be referred to as "perfluorocyclohexanecarboxamides." An exothermic reaction ensued. The mixture was then stirred overnight, washed with water, the organic layer dried over $MgSO_4$ and the product concentrated. Distillation gave 294.7 grams (72%) of the amide, boiling at 120-140 ° C/30 Torr. Infrared spectral analysis of the amide showed a major peak at 1640 $cm^{-1}$. GC/MS supported the major peak (89%) to be the 6-membered ring and having the following structure.

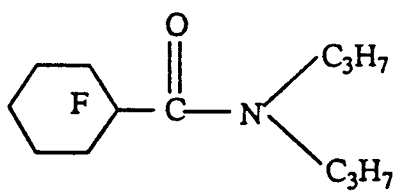

The ECF of the partially fluorinated starting material, N,N-dipropyl perfluorocyclohexanecarboxamide was carried out in a smaller Simons cell under similar conditions as Example 4. The crude perfluorinated liquid was distilled on a 6" Vigreux column, with the resulting distillate having a bp of 180-190 ° C. Treatment with aqueous KOH and redistillation gave a main cut, having a bp of 180-187 ° C. The crude perfluorinated liquids, contained approximately 79% of Class 1 compounds, with the remaining 15-20% being perfluorocyclohexanecarbonyl fluoride, which can be recycled to produce more feed material. Analysis indicates the following structure.

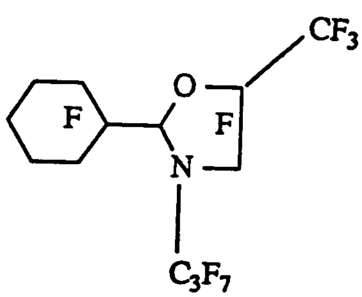

Example 6

A mixture of 129.5 g (1.0 mol) di-n-butylamine and 102 g (1.0 mol) triethylamine was dissolved in 250 mL of methylene chloride and treated slowly with 350 g of the 70-80 ° C bp fraction of the product of ECF of benzoyl chloride. The product was washed with water and distilled to 391.9 g (95%) at 115-123 ° C/3 Torr

and had the following structure.

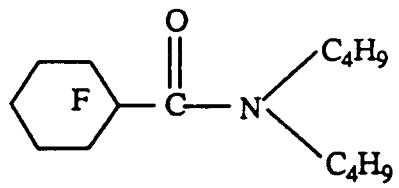

The ECF of N,N-dibutyl perfluorocyclohexane carboxamide was carried out at 30 to 40 psig and 50 ° C to 66 ° C and at 27 amps average current in a 1500cc Simons cell. Crude perfluorinated liquid was collected and contained 55% to 60% of desired $C_{15}$ Class 1 compounds including the following proposed structure. The major product had a bp in the range of 202-224 ° C (ASTM bp of 208 ° C). GC/MS supported the major peak (40-50%) to be a cyclized product having the formula $C_{15}F_{29}NO$.

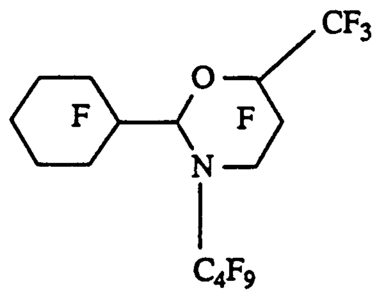

Example 7

The starting material, perfluoromethylcyclohexanecarbonyl fluoride was made by ECF of p-toluoyl chloride, and consisted of a mixture of isomers of the 5- and 6-membered rings. A mixture of 220 g each (2.2 mol each) of dipropylamine and triethylamine was treated with 760 g (2.0 mol) perfluoromethylcyclohexanecarbonyl fluoride. The resulting slurry was washed with water and the product (571.2 g) was distilled to a light orange liquid, with a bp of 145-150 ° C/43 Torr.

The ECF of N,N-dipropyl perfluoromethylcyclohexane carboxamide was carried out in a similar sized cell system at 30 psig control and 55 ° C and at 35 amps/ft current density and 6.0 volt average. Crude perfluorinated liquid was phase split from the HF-electrolyte at a rate equal to 85% theoretical based on current and contained about 60% of desired $C_{13}$ fluorocarbons. The perfluorinated liquid contained Class 1 compounds including the following proposed structure. The perfluorinated liquid had a bp in the range of 204-210 ° C. The liquid was then treated with aqueous KOH to give purified liquid with an ASTM bp 205 ° C.

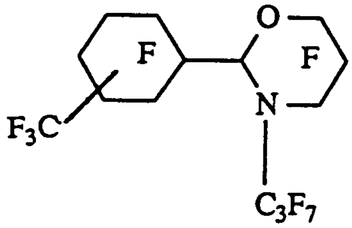

Example 8

The ECF of N,N-dipropyloctanamide was carried out in 1500cc Simons cell at 50 ° C to 55 ° C and 25 psig control at 6.1 volts average. Crude fluorocarbons were obtained at 70% of theoretical with a GC

indicated purity of 50% desired C14 fluorocarbons.

Fractionation, followed by treatment with aqueous KOH and redistillation produced a perfluorinated liquid with a bp in the range of 197-215°C (ASTM bp was 205°C). GC-MS analysis showed 78% of this fraction to have a molecular weight (MW) of 749 for the cyclized product $C_{14}F_{29}NO$. The analytical data indicates the presence of perfuorotetrahydrofuran and perfluorodihydropyran rings of Class 2 compounds. In addition, the data showed a small amount of product to have MW of 787 ($C_{14}F_{31}NO$), corresponding to the product of ring opening of the cyclic product and a small amount of product to have MW of 649, corresponding to the cyclic product minus $C_2F_4$.

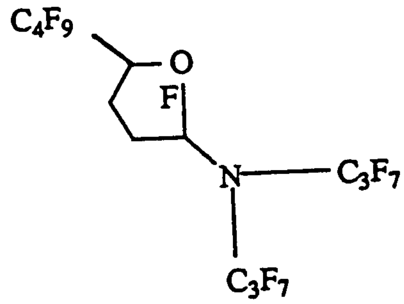

Example 9

The ECF of N,N-dipentyl perfluoromethylcyclohexanecarboxamide was carried out in a Simons cell at 30 to 35 psig control and at 55°C to 65°C and using 10% dimethyldisulfide. Crude perfluorinated liquid was obtained at 60-64% of theoretical, and contained about 55% of C18 desired product and 15% of perfluoro-methylcyclohane carbonyl fluoride, a starting material.

A portion of the crude ECF product was added to aqueous KOH. After the exothermic reaction was complete, the mixture was heated to 80-90°C for 18 hours. The lower phase was distilled under reduced pressure to a main cut, having a bp of 110-155°C/50-60 Torr. Subsequent redistillation at atmospheric pressure gave a fraction at 240-250°C containing Class 1 compounds, including the following proposed structure. The atmospheric boiling point was 246°C. GC-MS indicated that the main components had the desired m/e of 911 for $C_{18}F_{35}NO$.

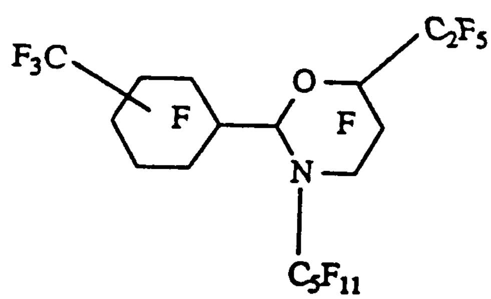

Example 10

The crude ECF product of dimethyl terephthalate was reacted with dipropylamine according to the procedure of Example 7. Distillation produced a diamide at 160-180°C/0.4 Torr and having a structure.

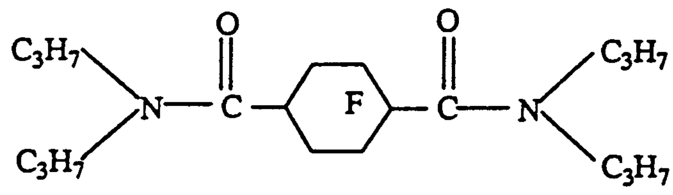

The ECF of N,N,N,N,-tetrapropyl perfluoro-1,4-cyclohexanedicarboxamide was carried out in a Simons cell at 20 psig and 45 ° C. Crude perfluorinated liquid was obtained at an average of 65% of theoretical. About 28% of perfluorocyclohexyl-1,4-dicarbonyl fluoride due to cleavage of the C-N bonds was recovered in the crude perfluorinated liquid.

Distillation of the crude ECF product, initially at atmospheric pressure yielded a liquid having a bp of 100-180 ° C, containing structure A, as supported by IR. Continued distillation of the residue under reduced pressure yielded product with a bp in the range of 180-230 ° C/50 Torr, postulated to contain structure B.

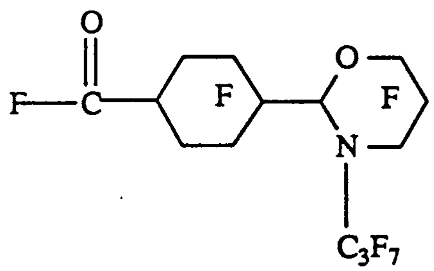

(A)

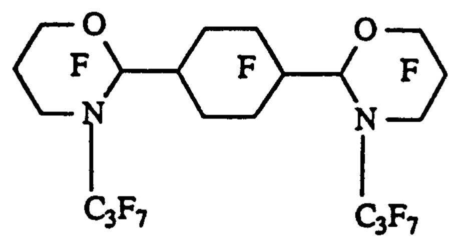

(B)

Example 11

The ECF of N,N,N,N-tetrapropyl-1,6-hexanedicarboxamide, dissolved in HF was carried out in a Simons cell of 1500cc capacity, wherein the starting material was fed continuously to the cell over a period of approximately 118 hours. The cell was run at 30 psig and 50 ° C at an average of 31.4 amps and 6.2 volts.

The crude fluorinated product was fractionated on a 3-plate Snyder column. After cut 4, the column was removed and the distillation was finished.

| Cut | Boiling Range |
|---|---|
| 1 | 40-70 ° C |
| 2 | 70-153 ° C |
| 3 | 155-175 ° C |
| 4 | 175-187 ° C |
| 5 | 220-247 ° C |
| 6 | 247-270 ° C |

Cuts 5 and 6 (bp range of 220-270 ° C) were cautiously added to a mixture of aqueous KOH and the resulting mixture was heated overnight at reflux. Distillation of the perfluorinated liquid layer gave material boiling at 245-255 ° C. The perfluorinated liquid contained Class 2 compounds, including the following proposed structure.

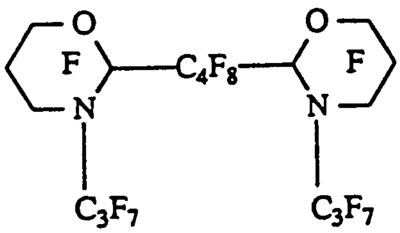

Cuts 3 and 4 (bp range of 155-187 °C) were mixed together. IR analysis showed a peak at 1890 $cm^{-1}$, indicating an acid fluoride. The combined cuts 3 and 4 were washed with excess methanolic $BF_3$ to convert the acid fluoride to the corresponding methyl ester. The resulting mixture was mixed with water and the lower layer was separated and distilled to a cut at 205-217 °C containing two major components. The IR spectrum contained a strong 1793 $cm^{-1}$ peak for the methyl ester. The GC/MS (m/e 639) supported a cyclized structure of Class 2 type compounds, including the following structure.

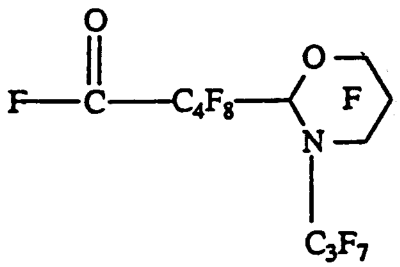

Example 12

The ECF of N,N-dipentyl-1-decanecarboxamide was carried out in a Simons cell having a capacity of 1500cc at an average of 28 amps and 7 volts. The temperature and pressure of the cell were approximately 60 °C and 35 psig, respectively. Approximately 10% DMDS was added to the starting feed material.

Distillation of the crude ECF product on a 6-plate Snyder column initially left a residue, having a boiling point greater than 170 °C. This residue was vacuum-distilled without a column to a main cut with a bp in the range of 90-200 °C/30 Torr.

A portion of the main cut was treated with a combined gas stream of 30 mL/min fluorine and 140 mL/min nitrogen in a stirred steel reactor at 200 °C for six hours to fluorinate residual hydride in the product. The resulting colorless liquid was far less reactive to base than before this treatment. Another portion of the main cut containing Class 2 compounds, including the following structure was treated with aqueous KOH and distilled. The GC/MS of the distillate supported a formula $C_{18}F_{37}NO$. The distillate had a bp in the range of 220-242 °C.

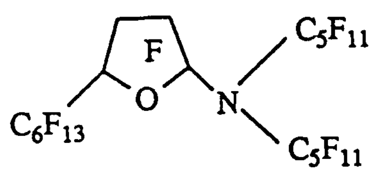

Example 13

The ECF of N,N-dibutyl-1-decanecarboxamide was carried out in a 1500 cc Simons cell at 10 to 30 psig and 35 to 60oC at 6.5 volts and 29.8 amps average. Some perfluorodecanoyl fluoride was found in the crude perfluorinated liquid recovered.

Treatment with aqueous KOH and distillation gave as the main component a colorless liquid containing

Class 2 compounds including the following proposed structure, boiling at 150-160 °C/25 Torr and an estimated pour point of -32 °C. The ASTM boiling point was 249 °C and the viscosity at 0 °C was 428 cs.

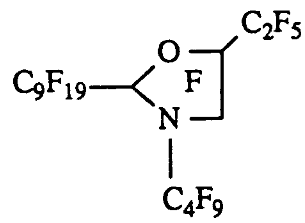

Example 14

The procedure described in Example 7 using di-n-pentylamine gave an 84% yield of N,N-dipentyl perfluorocyclohexanecarboxamide. The amide, a viscous liquid, distilled at 115-125 °C/0.2 Torr.

The ECF of N,N-dipentyl perfluorocyclohexanecarboxamide was carried out in a 1500cc Simons cell at 45 °C and 17 amps at 30 psig, running at an average of 6.5 volts.

The boiling point of the main product containing Class 1 compounds including the following proposed structure was approximately 224 °C. $^{19}F$ NMR was complex but provided evidence for the desired perfluorinated oxazolidine ring in approximately 35% concentration. The analogous oxazole was present as another major product.

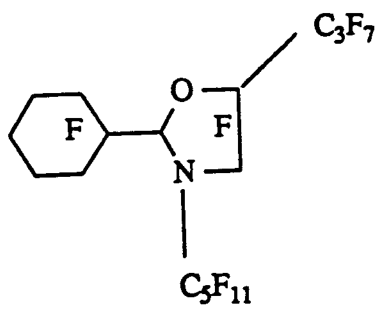

**Claims**

1. A perfluorinated liquid comprising one or a mixture of a perfluorinated, N-alkyl, aliphatic-substituted, saturated 1,3-oxazolidine and a perfluorinated N-alkyl, aliphatic-substituted, saturated 1,3-oxazine.

2. The liquid according to Claim 1, wherein said liquid is represented by one or a mixture of:

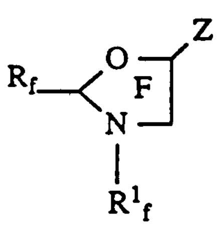

(I)

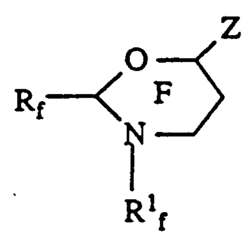

(II)

wherein $R_f$ is a saturated fluoroaliphatic group having one or more fully-fluorinated, saturated-aliphatic carbon atoms; the skeletal chain of $R_f$ can be straight, branched, or, if sufficiently large, cyclic

or contain a cyclic structure, said group can be composed of only carbon and fluorine, or it can include carbon, fluorine and a catenary divalent oxygen, or a caternary trivalent nitrogen, such hetero atom being a chemically stable linkage between the fluorocarbon portions of the fluoroaliphatic group;

Z is a lower fluoroalkyl having 1 to 8 carbon atoms or a fluorine atom;

$R^1_f$ attached to the depicted tertiary amino ring atom comprises a fully-fluorinated, saturated-aliphatic carbon atom, or a cyclic or acyclic group comprising a plurality of such carbon atoms, in a chain that can be a straight (normal) chain or a branched chain; and

further, $R^1_f$ and Z taken together can form a 5-, 6-, or 7-membered ring composed of carbon atoms only, or composed of carbon atoms and N or O hetero atoms, or $R^1_f$ and $R_f$ taken together can form a 5-, 6-, or 7-membered ring composed of carbon atoms only, or composed of carbon atoms and N or O hetero atoms.

**3.** The liquid according to Claim 2, wherein $R_f$ is a perfluorinated, saturated cycloaliphatic group have 4, 5, or 6 ring carbon atoms, such that a ring carbon atom is covalently bonded to the ring carbon atom positioned between the depicted oxygen and nitrogen.

**4.** A perfluorinated liquid comprising one or a mixture of a perfluorinated N-alkyl, aliphatic-substituted, saturated 1,3-oxazolidine, a perfluorinated N-alkyl, aliphatic-substituted, saturated 1,3-oxazine, a perfluorinated aliphatic-substituted, 2-dialkylaminotetrahydrofuran, and a perfluorinated aliphatic-substituted 2-dialkylaminodihydropyran.

**5.** The liquid according to Claim 4, wherein said liquid is represented by one or a mixture of:

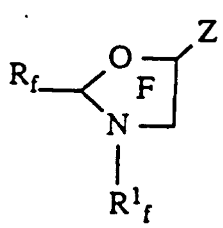

(I)

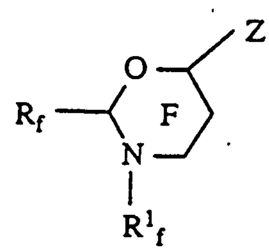

(II)

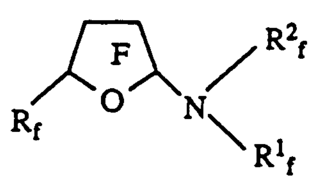

(III)

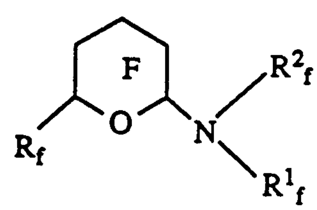

(IV)

wherein $R_f$ is a saturated fluoroaliphatic group having one or more fully-fluorinated, saturated-aliphatic carbon atoms, but not more than 20, such that the skeletal chain of $R_f$ can be straight, branched, or, if sufficiently large, cyclic or contain a cyclic structure, said group can be composed of only carbon and fluorine, or it can include carbon, fluorine and a catenary divalent oxygen, or a caternary trivalent nitrogen, such hetero atom being a chemically stable linkage between the fluorocarbon portions of the fluoroaliphatic group;

Z is a lower fluoroalkyl having 1 to 8 carbon atoms or a fluorine atom;

$R^1_f$ attached to the depicted tertiary amino ring atom comprises a fully-fluorinated, saturated-aliphatic carbon atom, or a cyclic or acyclic group comprising a plurality of such carbon atoms, in a chain that can be a straight (normal) chain or a branched chain; further, $R^1_f$ and Z taken together can form a 5-, 6-, or 7-membered ring composed of carbon atoms only, or composed of carbon atoms and N or O hetero atoms, or $R^1_f$ and $R_f$ taken together can form a 5-, 6-, or 7-membered ring composed of carbon atoms only, or composed of carbon atoms and N or O hetero atoms; and

$R^2_f$ is defined as $R^1_f$ and may be the same or different, with the proviso that if either $R^1_f$ or $R^2_f$ is a

trifluoromethyl group, the sum of carbon atoms of $R^1_f$ and $R^2_f$ comprise at least 3 carbon atoms, or $R^1_f$ and $R^2_f$ taken together can form a 5-, 6-, or 7-membered ring composed of carbon atoms only, or composed of carbon atoms and N or O hetero atoms.

**6.** The liquid according to Claim 5, wherein $R_f$ is a perfluorinated, saturated cycloaliphatic group have 4, 5, or 6 ring carbon atoms, such that a ring carbon atom is covalently bonded to the ring carbon atom positioned between the depicted oxygen and nitrogen.

**7.** A process comprising electrolyzing in an electrolytic cell a solution of liquid, anhydrous hydrogen fluoride containing a liquid comprising a perfluorinatable N,N-dialkyl carboxamide.

**8.** The process according to Claim 7, wherein said perfluorinatable N,N-dialkyl carboxamide is represented by:

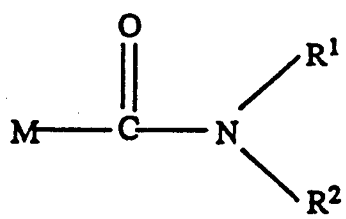

M is either R or

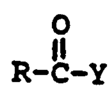

wherein R is an acyclic, cyclic, aromatic, fluoroaliphatic, fluorocycloaliphatic group having one or more fluorinatable carbon atoms and is composed of only carbon, hydrogen, and fluorine, or carbon and hydrogen and can include caternary divalent oxygen or caternary trivalent nitrogen, such hetero atoms being a chemically stable linkage between the carbon portion of the R group; and

Y is selected from

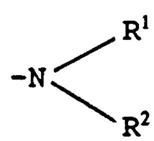 ,

-X, -OH and $-OR^3$

wherein X is a halide, preferably fluorine, and

$R^3$ is a lower alkyl having 1 to 8 carbon atoms; and

$R^1$ or $R^2$ is an alkyl group with at least 2 carbon atoms and generally not more than 8 carbons, $R^1$ and $R^2$ may be the same or different, with a first proviso that if either $R^1$ or $R^2$ is a trifluoromethyl group, the sum of the carbon atoms of $R^1$ and $R^2$ comprise at least 3 carbon atoms and a second proviso that if either $R^1$ or $R^2$ is an aromatic group, the other is an alkyl group having 2 or more carbon atoms, or $R^1$ and $R^2$ taken together can form a 5-, 6-, or 7-membered ring composed of carbon atoms only, or composed of carbon atoms and N or O hetero atoms.

**9.** The process according to Claim 8, wherein said perfluorinatable N,N-dialkyl carboxamide is partially fluorinated and produces the liquid according to Claim 3.

**10.** The liquid according to Claim 2, wherein said $R_f$ is a perfluorinated, saturated, cycloaliphatic group composed of a 6-membered ring; Z is selected from the group consisting of fluorine, $-CF_3$, $-C_2H_5$, and

$-C_3F_7$; and $R^1_f$ is selected from the group consisting of $-CF_3$, $-C_2F_5$, $-C_3F_7$, $-C_4F_9$, and $-C_5F_{11}$.

**11.** The liquid according to Claim 2, wherein said liquid is made by electrolyzing in an electrolytic cell a solution of liquid, anhydrous fluoride containing a liquid comprising a partially fluorinated fluorinatable N,N-dialkyl carboxamide.

**12.** The liquid according to Claim 5, wherein said liquid is made by electrolyzing in an electrolytic cell a solution of liquid, anhydrous fluoride containing a liquid comprising a fluorinatable N,N-dialkyl carboxamide.